# EUROPÄISCHE PATENTSCHRIFT

(11) **EP 1 098 611 B1**
(45) Veröffentlichungstag und Bekanntmachung des Hinweises auf die Patenterteilung: **12.07.2006**
(21) Anmeldenummer: 98967162.3
(22) Anmeldetag: 25.05.1998
(51) Int. Cl.: A61F 2/34

(54) **HÜFTPFANNENPROTHESE**
COTYLOID CAVITY PROSTHESIS
PROTHESE DE LA CAVITE COTYLOIDE

(43) Veröffentlichungstag der Anmeldung: 16.05.2001
(73) Patentinhaber: WALDEMAR LINK GmbH & Co. KG, 22339 Hamburg (DE)
(72) Erfinder: KELLER, Arnold, D-23863 Kayhude (DE)
(74) Vertreter: Glawe, Delfs, Moll
(86) Internationale Anmeldenummer: PCT/EP1998/003076
(87) Internationale Veröffentlichungsnummer: WO 1999/060955

(56) Entgegenhaltungen:
- EP-A- 0 190 093
- US-A- 5 133 764
- US-A- 5 507 828

## Beschreibung

Die Erfindung betrifft eine Hüftpfannenprothese, deren Lagereinsatz in einer Außenschale durch eine Rasteinrichtung gehalten ist, die eine Hinterschneidungsfläche an der *Außenschale* und eine dahintergreifende, *vom Lagereinsatz schräg nach außen zur Öffnungsseite der Prothese hin stehende* elastische Rastlippe umfaßt.

Bei bekannten Hüftpfannenprothesen dieser Art ist der aus Polyethylen bestehende Lagereinsatz mit einer elastischen Haltelippe versehen, die hinter Vorsprünge der Außenschale einschnappt *(US-A-5,133,764)*. Damit der Lagereinsatz fest in der Außenschale sitzt, ist man bemüht, jegliches Spiel zwischen der Lippe und der Hinterschneidungsfläche zu vermeiden. Da aber Herstellungstoleranzen berücksichtigt werden müssen, läßt sich dieses Ziel in der Praxis nicht erreichen.

Der Erfindung liegt die Aufgabe zugrunde, eine Hüftpfannenprothese der eingangs und im Oberbegriff des Anspruchs 1 genannten Art zu schaffen, die eine spielfreie und dennoch toleranzausgleichende Halterung des Einsatzes in der Schale ermöglicht.

Die erfindungsgemäße Lösung liegt in den Merkmalen des Anspruchs 1. Im montierten Zustand *schließt die Hinterschneidungsfläche mit der Montageeinrichtung im Berührungspunkt einen kleineren Winkel ein als die Tangente an die Verformungslinie der Lippenspitze im Berührungspunkt mit der Hinterschneidungsfläche, und* die Lippe des Lagereinsatzes liegt unter Biegeverformung mit ihrer Spitze *selbsthemmend* an der Hinterschneidungsfläche an.

*Die Verformungslinie* ist diejenige Kurve, auf der sich die Lippenspitze bei einer Biegeverformung der Lippe unter der von der Hinterschneidungsfläche auf ihre Spitze ausgeübten Kraft bewegt. Diese Kurve hat im Berührungspunkt die Richtung, in der sich die von der Hinterschneidungsfläche festgehaitene Lippenspitze weiter nach außen bewegen würde, wenn die Hinterschneidungsfläche nicht vorhanden wäre. Als Bezugsrichtung für die Richtung der Hinterschneidungsfläche und der Verformungslinie der Lippenspitze wurde oben die Montagerichtung des Einsatzes genannt. Es ist an sich gleichgültig, welche Bezugsrichtung für den Vergleich dieser beiden Richtungen gewählt wird. Einfachheitshalber wird für diese Zwecke im Anspruch die Mittelachse der Prothese genannt, wobei es nicht darauf ankommt, wie die Richtung dieser Mittelachse bei komplexen Prothesenformen ermittelt wird, da es auf deren genaue Richtung nicht ankommt, sofern sie nur festgelegt ist.

*Unter der Kraft der Biegeverformung der Lippe übt die Lippenspitze in radialer und insbesondere in axialer Richtung* eine elastische Kraft auf die Hinterschneidungsfläche aus, deren Reaktionskraft den Lagereinsatz weiter in die Außenschale hineindrückt, bis sie eine spielfreie Endstellung an einem Anschlag erreicht, der beispielsweise durch den Boden der Hüftpfanne gebildet ist. *Dabei* wirkt die Lippe mit der Hinterschneidungsfläche selbsthemmend zusammen. Darunter ist zu verstehen, daß die geometrischen Verhältnisse des Zusammenwirkens von Lippe und Hinterschneidungsfläche so gewählt sind, daß unter einer den Einsatz aus der Außenschale zu lösen trachtenden Krafteinwirkung die Lippe nicht elastisch zusammengedrückt an der Hinterschneidungsfläche entlang nach außen gleiten und herausschnappen kann, sondern durch Reibung festgehalten wird.

Damit dieser Montagezustand jedenfalls zustande kommt, sind die Abmessungen der Hinterschneidungsfläche und der Lippe so gewählt, daß auch bei größten Toleranzabweichungen die Lippe , jedenfalls auf die Hinterschneidungsfläche trifft. Diese Bedingung ist leicht einzuhalten, da die Hinterschneidungsfläche mit einer axialen Erstreckung ausgeführt werden kann, die größer ist als alle vorkommenden Maßtoleranzen in dieser Richtung. Ferner ist vorgesehen, daß sämtliche Bereiche der Hinterschneidungsfläche, die für einen Kontakt mit der Lippenspitze vorgesehen sind, einen Durchmesser haben, der geringer ist als der entspannte Durchmesser der Lippenspitze.

*Bei bekannten Prothesen ähnlicher Art steht die Lippe flach nach innen von der Öffnungsseite der Prothese weg (US-A-5,507,828) und drückt radial gegen die Außenschale; die Lippe wirkt mit der Auflagefläche nicht selbsthemmend zusammen und kann unter einer axial zur Öffnungsseite der Prothese wirkenden Kraft nach außen gleiten und herausschnappen.*

Die Hinterschneidungsfläche, mit der die Lippe des Lagereinsatzes zusammenwirkt, kann Teil eines Vorsprungs, beispielsweise eines Wulstes, oder eines Rezesses sein, beispielsweise einer Nut, insbesondere einer Ringnut. Der Rezeß oder der Vorsprung können entlang des gesamten Umfangs oder nur in Teilen davon vorgesehen sein, entsprechend kann die Lippe als Ringlippe ausgeführt sein oder aus mehreren Segmenten bestehen, die beispielsweise fingerartig mit als Nasen ausgebildeten Vorsprüngen zusammenwirken.

Bei den vorstehenden Ausführungen ist - wie üblich - angenommen worden, daß die Lippe an dem Lagereinsatz und die Hinterschneidung an der Hüftpfanne angeordnet ist, es kann jedoch auch umgekehrt die Lippe an der Hüftpfanne und die Hinterschneidung an dem Lagereinsatz vorgesehen sein, wenn die Materialeigenschaften dies zulassen.

Die Erfindung wird im folgenden näher unter Bezugnahme auf die Zeichnung erläutert, die ein vorteilhaftes Ausführungsbeispiel veranschaulicht. Darin zeigen:
- Fig. 1: einen Längsschnitt durch eine Hüftpfanne in zwei verschiedenen Phasen der Montage,
- Fig. 2: einen vergrößerten Teilschnitt, der die bei der Montage sich ergebenden Verformungszustände der Lippe veranschaulicht,
- Fig. 3: einen der Fig. 1 entsprechenden Längsschnitt im vollständig montierten Zustand,
- Fig. 4: einen vergrößerten Teilschnitt der Rasteinrichtung im montierten Zustand und
- Fig. 5: eine Darstellung der geometrischen Verhältnisse im Berührungspunkt der Lippe.
Die Hüftpfannenprothese umfaßt eine Außenschale 1 und einen Lagereinsatz 2. Die Außenschale besteht beispielsweise aus Metall und ist äußerlich in beliebiger Weise zur Implantation im Hüftknochen gestaltet.

Der Einsatz 2 besteht aus Polyethylen oder einem anderen lagergeeigneten Werkstoff. Da dieser im vorliegenden Fall die Lippe bildet, soll er elastisch verformbar sein. Die Innengestalt der Außenschale 1 entspricht der Außengestalt des Einsatzes 2 in solcher weise, daß der Einsatz durch die Außenschale hinreichend gestützt wird. Im dargestellten Beispiel sind sie sphärisch und liegen vollflächig aneinander an. Diese Anlage soll möglichst spielfrei gewährleistet sein.

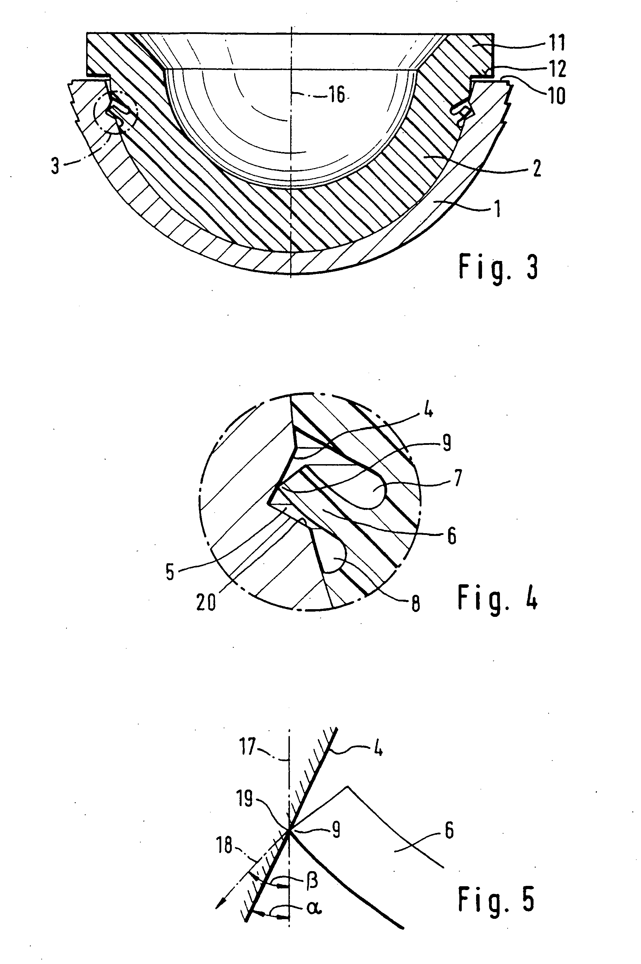

Der Einsatz 2 wird in der Außenschale 1 durch eine Rasteinrichtung gehalten, die in Fig. 3 allgemein mit der Bezugsziffer 3 versehen ist und in Fig. 4 näher dargestellt ist. Sie besteht aus einer Hinterschneidungsfläche 4, die von einer Nut 5 in der Innenfläche der Außenschale gebildet ist, und einer Lippe 6, die umlaufend von dem Einsatz vorspringt und mit der Hinterschneidungsfläche 4 zusammenwirkt. Damit die Lippe 6 hinreichende Länge hat, um eine geeignete Nachgiebigkeit entwickeln zu können, wird sie von zwei Nuten 7,8 eingefaßt. Sie weist eine Spitze 9 auf, die zum Zusammenwirken mit der Hinterschneidungsfläche 4 bestimmt ist. Die Rasteinrichtung 3 ist nahe dem freien Rand 10 der Außenschale angeordnet. Der Einsatz 2 weist einen radial nach außen vorragenden Randflansch 11 auf, der nicht nur der Verstärkung dient, sondern auch dem Operateur beim Einsetzen des Einsatzes in die Außenschale 1 behilflich ist. Seine Rückfläche 12 wirkt mit geringem Spiel mit der Stirnfläche des Außenschalenrandes 10 zusammen und erzwingt dadurch im Endstadium des Einsetzens eine Stellung des Einsatzes, die dessen gewünschter Endlage nahekommt und Gewähr dafür gibt, daß die Lippe 6 in die Nut 5 eingreift.

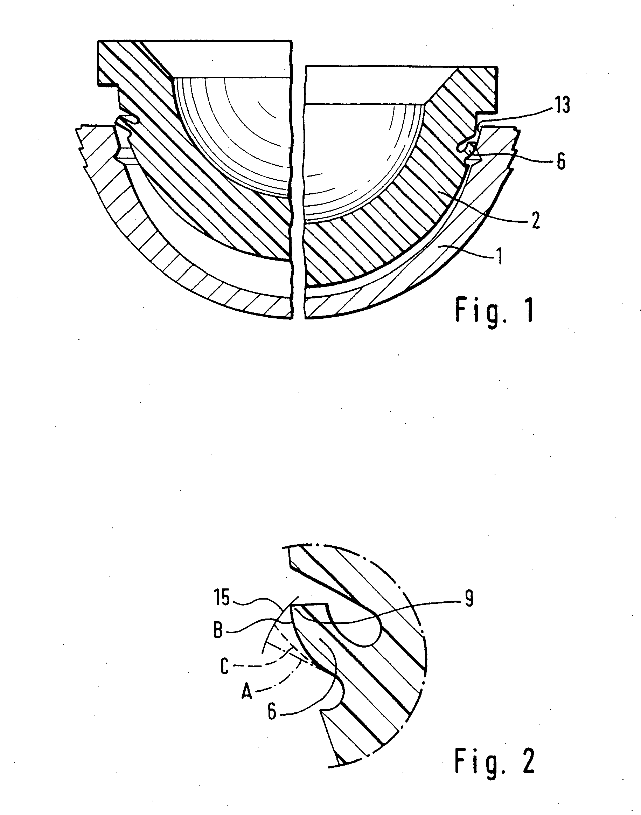

Die Lippe 6 ist im entspannten, ursprünglich gefertigten Zustand (s. Fig. 1 links) im Querschnitt gerade. Sie bildet insgesamt einen Kegel, dessen Spitze dem Boden der Prothese zugewandt ist. Die Lippe steht demzufolge schräg nach außen zur Öffnungsseite der Prothese hin. In Fig. 2 ist die äußere Lippenflanke im entspannten, geraden Zustand mit der Bezeichnung A versehen.

Zu Beginn der Montage passiert die Lippe 6 einen Bereich 13, der einen wesentlich geringeren Durchmesser hat als die Lippenspitze 9. Die Lippe wird daher kräftig verbogen, wie es in Fig. 1 rechts gezeigt ist. Diese stark gebogene Stellung ist in Fig. 2 mit der Bezeichnung B versehen.

Sobald die Lippe die Nut 5 erreicht, rastet sie in diese ein, indem sie radial aufspringt, bis sie an der Hinterschneidungsfläche 4 anliegt, wie es in Fig. 4 dargestellt ist. Dieser Biegezustand der Lippe ist in Fig. 2 mit dem Buchstaben C gekennzeichnet.

In Fig. 2 ist auch die Linie 15 gezeichnet, auf der die Lippenspitze 9 in den unterschiedlichen Verformungszuständen liegt. Diese Linie wird im Anspruch als Verformungslinie der Lippenspitze bezeichnet. Die Hinterschneidungsfläche 4 ist so angeordnet, daß die Verformungslinie 15 in jedem Fall, auch bei Berücksichtigung der größtmöglichen Maßabweichungen des Lagereinsatzes und der Außenschale, diese Hinterschneidungsfläche trifft. Ferner ist deren Radius im Verhältnis zum Radius der Lippenspitze so gewählt, daß jedenfalls derjenige Teil der Hinterschneidungsfläche 4, der von der Lippenspitze getroffen werden kann, einen kleineren Durchmesser als die Lippenspitze hat. Dadurch ist sichergestellt, daß im montierten Zustand die Lippenspitze stets unter Biegeverformung der Lippe an der Hinterschneidungsfläche 4 anliegt.

Bei dieser Anlage ergibt sich aus der elastischen Verformungskraft der Lippe eine Reaktionskraft, die den Lagereinsatz weiter in die Außenschale hineinzudrängen versucht. Dadurch werden etwa noch verbleibende Spielräume geschlossen. Spätestens geschieht dies bei mechanischer Beanspruchung des Lagereinsatzes.

Das Zusammenwirken der Lippenspitze 9 mit der Hinterschneidungsfläche 4 ist dann gesichert, wenn der in Fig. 4 mit der Bezeichnung α versehene Winkel, den die Hinterschneidungsfläche 4 mit einer parallel zur Mittelachse 16 der Prothese verlaufenden Bezugslinie 17 einschließt, geringer ist als der Winkel β zwischen dieser Bezugslinie und der Richtung 18, die der Tangente an die Verformungslinie 15 der Lippenspitze im Berührungspunkt 19 entspricht. Es handelt sich dabei um die Richtung, in der sich die an der Hinterschneidungsfläche 4 anliegende Lippenspitze 9 weiterbewegen möchte.

Damit die Lippenspitze an der Hinterschneidungsfläche 4 anliegt, sollte die innere Begrenzung 20 der Nut 5 stets in hinreichender Entfernung von der Lippe 6 liegen.

Wenn die Winkel, unter denen die Lippe 6 und die Hinterschneidungsfläche 4 zusammenwirken, so gewählt sind, daß Selbsthemmung stattfindet oder wenigstens die Kraft, unter der die Lippe 6 an der Hinterschneidungsfläche 4 in der Montagerichtung zurückgleiten kann, größer ist als die Lösekräfte, die man im Einsatz der Prothese erwarten muß, ist Gewähr dafür gegeben, daß der Einsatz 2 stets spielfrei in der Außenschale 1 gehalten ist. Dafür ist es nicht erforderlich, daß die Lippe 6 die unmittelbar nach der Montage noch wirkende elastische Kraft auf Dauer beibehält. Vielmehr ist bei Polyethylen und ähnlichen Werkstoffen zu erwarten, daß diese Kraft allmählich nachläßt. Die Haltewirkung wird dadurch aber nicht oder nicht wesentlich beeinträchtigt.

Der Winkel α liegt zweckmäßigerweise zwischen 15° und 40°, weiter vorzugsweise zwischen 20° und 30°. Die Differenz zwischen den Winkeln α und β ist zweckmäßigerweise nicht größer als 30° und liegt mit Vorteil in der Größenordnung von 20°.

## Patentansprüche

1. Hüftpfannenprothese, deren Lagereinsatz (2) in einer Außenschale (1) durch eine Rasteinrichtung (3) gehalten ist, die eine Hinterschneidungsfläche (4) an der *Außenschale (1) und* eine dahintergreifende, *vom Lagereinsatz (2) schräg nach außen zur Öffnungsseite der Prothese hin stehende* Rastlippe (6) umfaßt, **dadurch gekennzeichnet, daß** die *Hinterschneidungsfläche (4) mit der Mittelachse (16) der Prothese einen kleineren Winkel (α) einschließt als die Tangente (18) an die Verformungslinie (15)* der *Lippenspitze im Berührungspunkt (19) mit der Hinterschneidungsfläche, und daß die Lippenspitze (9) unter der Kraft der* Biegeverformung der Lippe (6) *selbsthemmend* an der Hinterschneidungsfläche (4) anliegt.

2. Hüftpfannenprothese nach Anspruch 1, **dadurch gekennzeichnet, daß** die Hinterschneidungsfläche (4) *durch eine Nut (5) gebildet ist*.

## Claims

1. Cotyloid cavity prosthesis, whose bearing insert (2) is held in an outer shell (1) by means of a locking device (3) which comprises an undercut surface (4) on the outer shell (1) and, engaging behind it, a locking lip (6) which projects obliquely outwards from the bearing insert (2) towards the open side of the prosthesis, **characterized in that** the undercut surface (4) forms, with the centre axis (16) of the prosthesis, a smaller angle (α) than the tangent (18) on the deformation line (15) of the tip of the lip at the contact point (19) with the undercut surface, and **in that** the tip (9) of the lip, under the force of the bending deformation of the lip (6), rests in a self-locking manner on the undercut surface (4).

2. Cotyloid cavity prosthesis according to Claim 1, **characterized in that** the undercut surface (4) is formed by a groove (5).

## Revendications

1. Prothèse de cavité cotyloïde, dont la garniture de palier (2) est maintenue dans une coquille extérieure (1) par un dispositif à cran (3) qui comporte une surface en contre-dépouille (4) sur la coquille extérieure (1) ainsi qu'une lèvre d'encliquetage (6) s'y accrochant par derrière, s'étendant depuis la garniture de palier (2) obliquement vers l'extérieur en direction du côté d'ouverture de la prothèse, **caractérisée en ce que** la surface en contre-dépouille (4) délimite avec l'axe médian (16) de la prothèse un angle (α) plus petit que celui de la tangente (18) avec la ligne de déformation (15) de la pointe de la lèvre au point de contact (19) avec la surface en contre-dépouille et **en ce que** la pointe de la lèvre (9) repose, sous la force de la déformation de la lèvre (6), retenu de lui-même, contre la surface en contre-dépouille (4).

2. Prothèse de cavité cotyloïde selon la revendication 1, **caractérisée en ce que** la surface en contre-dépouille (4)est formé par une rainure (5).
